# EUROPEAN PATENT APPLICATION

(11) **EP 4 763 973 A1**
(43) Date of publication of application: **24.06.2026**
(21) Application number: 24854232.6
(22) Date of filing: 16.08.2024
(51) Int. Cl.: C12N 1/21, C12N 1/20, C12N 15/09, C12N 15/53, C12P 1/04, C12Q 1/02

(54) **BACTERIUM HAVING HIGH CARBON DIOXIDE YIELD RELATIVE TO CARBON AND METABOLIC ENZYME GENE-DISRUPTED STRAIN OF SAME**

(30) Priority: 17.08.2023 JP 2023132996
(71) Applicant: Nagase & Co., Ltd., Osaka 550-8668 (JP)
(72) Inventor: TSUGE Yota, Kanazawa-shi, Ishikawa 920-1192 (JP); YAMAMOTO Shogo, Kobe-shi, Hyogo 651-2241 (JP)
(74) Representative: Klöckner, Christoph
(86) International application number: PCT/JP2024/029130
(87) International publication number: WO 2025/037644

(57) **Abstract**

The present disclosure provides a bacterium with a high yield relative to carbon of carbon dioxide and a metabolic enzyme gene-disrupted strain of the bacterium. The present disclosure also provides a method for isolating a bacterium with a high yield relative to carbon of carbon dioxide. The present disclosure also provides a method for producing, a method for cultivating, and a method for using the metabolic enzyme gene-disrupted strain of the bacterium. According to the present disclosure, by disrupting a gene encoding an enzyme of a metabolic reaction producing carbon dioxide in a bacterium with a high yield relative to carbon of carbon dioxide, for example, environmental load due to cultivation of the bacterium can be reduced, and/or bacterial cells can be changed to a state more suitable for substance production.

## Description

### TECHNICAL FIELD

The present disclosure relates to a bacterium with a high yield relative to carbon of carbon dioxide and a metabolic enzyme gene-disrupted strain of the bacterium. The present disclosure also relates to a method for isolating a bacterium with a high yield relative to carbon of carbon dioxide. The present disclosure also relates to a method for producing, a method for cultivating, and a method for using the metabolic enzyme gene-disrupted strain of the bacterium.

### BACKGROUND ART

In lysine production, reducing the activity of isocitrate dehydrogenase (ICD) of coryneform bacteria is disclosed (Patent Documents 1 to 5, Non-Patent Document 4). It is disclosed that deficiency of the isocitrate dehydrogenase gene (icd) of *Escherichia coli* causes a decrease in growth ability and glucose consumption (Non-Patent Documents 1 to 2), and it is disclosed that icd disruption in coryneform bacteria results in glutamate auxotrophy (Non-Patent Document 3). In Non-Patent Documents 4 to 9, various studies on disrupting metabolic enzymes are disclosed.

### PRIOR ART

### PATENT DOCUMENTS

[Patent Document 1] JP2015-154768A
[Patent Document 2] JP2013-533740A
[Patent Document 3] JP2011-518571A
[Patent Document 4] JP5719434B
[Patent Document 5] JP5395893B

### NON-PATENT DOCUMENTS

### [Non-Patent Documents]

[Non-Patent Document 1] Appl. Microbiol. Biotechnol., 2004, 65: 84-96
[Non-Patent Document 2] FEMS Microbiol. Lett., 2019, 366
[Non-Patent Document 3] J. Bacteriol., 1995, 177(3): 774-782
[Non-Patent Document 4] Appl. Environ. Microbiol., 2009. 75(24): 7866-7869 [Non-Patent Document 5] Microbial. Biotechnol., 2019. 12(5): 932-945
[Non-Patent Document 6] J.bacteriol., (2014). 3045-3057
[Non-Patent Document 7] BMC Biotechnol., (2016) 16:52
[Non-Patent Document 8] Metabolomics, (2022) 18:56
[Non-Patent Document 9] Appl. Environ. Microbiol., 2012. 78(19): 6975-6986

### SUMMARY OF THE INVENTION

The present disclosure provides a bacterium with a high yield relative to carbon of carbon dioxide and a gene-modified strain (particularly a metabolic enzyme gene-disrupted strain) of the bacterium. The present disclosure also provides a method for isolating a bacterium with a high yield relative to carbon of carbon dioxide. The present disclosure also provides a method for producing, a method for cultivating, and a method for using the bacterium (particularly the metabolic enzyme gene-disrupted strain).

According to the present disclosure, for example, the following inventions are provided.
(1) A metabolic enzyme gene-disrupted strain of an actinomycete (preferably an actinomycete of the genus Streptomyces) whose yield relative to carbon of carbon dioxide (CO₂) exceeds 50% in at least a logarithmic growth phase or a stationary phase in aerobic culture in a medium containing a sufficient amount of a carbon source and other nutrients, wherein at least one of genes encoding an enzyme responsible for a metabolic reaction producing CO₂ is disrupted, and the yield of CO₂ relative to carbon is reduced relative to a control actinomycete of the same species in which none of the genes are disrupted.
(2) The metabolic enzyme gene-disrupted strain according to the above (1), wherein the enzyme responsible for a metabolic reaction producing CO₂ is an enzyme of any one or more metabolic pathways selected from the group consisting of glycolysis, pentose phosphate pathway, and citric acid cycle.
(3) The metabolic enzyme gene-disrupted strain according to the above (1) or (2), wherein one or more selected from the group consisting of phosphogluconate dehydrogenase, pyruvate dehydrogenase, phosphoenolpyruvate carboxykinase, malate dehydrogenase, isocitrate dehydrogenase, dihydrolipoyl transacetylase, dihydrolipoyl dehydrogenase, and α-ketoglutarate dehydrogenase are disrupted.
(4) The metabolic enzyme gene-disrupted strain according to any one of the above (1) to (3), wherein in aerobic culture in the presence of a sufficient amount of a carbon source, compared to the control actinomycete, its growth ability is equivalent, or it does not show a decrease in growth ability by 50% or more.
(5) The metabolic enzyme gene-disrupted strain according to any one of the above (1) to (4), wherein one or more genes consisting of at least isocitrate dehydrogenase, phosphogluconate dehydrogenase, pyruvate dehydrogenase, pyruvate carboxykinase, and malate dehydrogenase are disrupted.
(5A) The metabolic enzyme gene-disrupted strain according to any one of the above (1) to (4), wherein at least isocitrate dehydrogenase is disrupted.
(5B) The metabolic enzyme gene-disrupted strain according to any one of the above (1) to (4), wherein at least phosphogluconate dehydrogenase is disrupted.
(5C) The metabolic enzyme gene-disrupted strain according to any one of the above (1) to (4), wherein at least malate dehydrogenase is disrupted.
(5D) The metabolic enzyme gene-disrupted strain according to any one of the above (1) to (4), wherein at least pyruvate carboxykinase is disrupted.
(6) The metabolic enzyme gene-disrupted strain according to any one of the above (1) to (5), wherein the actinomycete is of the genus Streptomyces.
(7) A composition comprising the metabolic enzyme gene-disrupted strain according to any one of the above (1) to (6), for use in producing a metabolite.
(8) A composition comprising the metabolic enzyme gene-disrupted strain according to any one of the above (1) to (6), for use in producing a metabolite derived from the citric acid cycle.
(9) A method for cultivating a metabolic enzyme gene-disrupted strain of an actinomycete,
   wherein
   the metabolic enzyme gene-disrupted strain comprises the metabolic enzyme gene-disrupted strain according to any one of the above (1) to (6), and
   the method comprises cultivating the metabolic enzyme gene-disrupted strain in a medium containing a sufficient amount of a carbon source and other nutrients under aerobic conditions.
(10) The method according to the above (9), wherein the cultivation comprises cultivating the metabolic enzyme gene-disrupted strain in a logarithmic growth phase, and cultivating the metabolic enzyme gene-disrupted strain in a stationary phase.
(11) A method for producing a metabolite using a metabolic enzyme gene-disrupted strain of an actinomycete, comprising:
   cultivating a metabolic enzyme gene-disrupted strain, wherein
   the metabolic enzyme gene-disrupted strain comprises the metabolic enzyme gene-disrupted strain according to any one of the above (1) to (6),
   the method comprises cultivating the metabolic enzyme gene-disrupted strain in a medium containing a sufficient amount of a carbon source and other nutrients under aerobic conditions to cause the metabolic enzyme gene-disrupted strain to produce the metabolite; and
   recovering the metabolite.
(12) The method according to the above (11), wherein the metabolite is derived from the citric acid cycle.
(13) A method for selecting an actinomycete, comprising:
   providing an isolated actinomycete;
   measuring a yield relative to carbon of carbon dioxide of the actinomycete in a logarithmic growth phase or a stationary phase in aerobic culture in a medium containing a sufficient amount of a carbon source and other nutrients; and
   selecting or obtaining an actinomycete whose yield relative to carbon exceeds 50%.
(14) A method for genetically modifying an actinomycete to obtain a genetically modified actinomycete, comprising:
   providing an isolated actinomycete;
   measuring a yield relative to carbon of carbon dioxide of the actinomycete in a logarithmic growth phase or a stationary phase in aerobic culture in a medium containing a sufficient amount of a carbon source and other nutrients;
   selecting or obtaining an actinomycete whose yield relative to carbon exceeds 50%;
   disrupting by genetic modification at least one of genes encoding an enzyme responsible for a metabolic reaction producing carbon dioxide of the obtained actinomycete; and obtaining the genetically modified actinomycete.

### BRIEF DESCRIPTION OF THE DRAWINGS

[FIG. 1] FIG. 1 shows metabolic pathways including glycolysis, pentose phosphate pathway, and TCA cycle.
[FIG. 2] FIG. 2 shows optical density (OD600), dry cell weight, and glucose consumption amount after the start of main culture of a wild-type strain (WT) and a Δicd strain of *Streptomyces lividans.*
[FIG. 3] FIG. 3 shows the production amount of undecylprodigiosin (Udp) by the wild-type strain (WT) and the Δicd strain of *Streptomyces lividans.*
[FIG. 4] FIG. 4 shows a synthetic pathway of Udp.
[FIG. 5] FIG. 5 shows optical density (OD600), dry cell weight, and glucose consumption amount after the start of main culture of a deficient strain (ΔpckA) of a gene (*pckA*) encoding an enzyme that produces phosphoenolpyruvate from oxaloacetate, a deficient strain (ΔmaeA) of a gene (*maeA*) encoding an enzyme that produces pyruvate from malate, and a deficient strain (Δgnd) of a gene (*gnd*) encoding an enzyme that produces ribulose-5-phosphate from 6-phosphogluconate of *Streptomyces lividans.*
[FIG. 6] FIG. 6 shows the production amount of undecylprodigiosin (Udp) by the ΔpckA strain.

### DESCRIPTION OF THE EMBODIMENTS

### <Definition of Terms>

In the present specification, "about" means a range within +/- 10%, +/- 5%, or +/- 1% of the numerical value that follows.

In the present specification, an "aerobe" is a microorganism equipped with a mechanism for consuming oxygen to produce energy. An aerobe utilizes oxygen to obtain energy from sugar and lipid and the like. Examples of the aerobe include facultative aerobes and obligate aerobes. A facultative aerobe can utilize oxygen but can also produce energy anaerobically. In the present specification, the aerobe may include microorganisms classified as anaerobes such as facultative anaerobes as long as they satisfy the above definition. Examples of the facultative aerobe include yeasts, bacteria, and molds. The aerobe in which a metabolic enzyme has been disrupted has been established as a strain. Therefore, the aerobe having a disrupted metabolic enzyme is sometimes simply referred to as a metabolic enzyme gene-disrupted strain in the present specification.

In the present specification, an "actinomycete" is a bacterium belonging to the phylum Actinobacteria (Actinomycetes). Examples of the actinomycete include, but are not particularly limited to, actinomycetes of the genus Streptomyces, the genus Actinomyces, the genus Mycobacterium, and the genus Corynebacterium. An actinomycete is a facultative aerobic bacterium and can produce energy under anaerobic conditions, but when utilized for substance production, it is cultivated under aerobic conditions. Representative examples of the actinomycete of the genus Streptomyces include *Streptomyces lividans, Streptomyces violaceoruber, Streptomyces coelicolor, Streptomyces avermitilis, Streptomyces griseus, Streptomyces rapamycinicus, Streptomyces kanamyceticus, Streptomyces peucetius, Streptomyces galilaeus, Streptomyces purvulus, Streptomyces albus, Streptomyces albulus, and the like.* Also, examples of the actinomycete of the genus Streptomyces include *S. albaduncus, S. albiaxialis, S. albidochromogenes, S. albidoflavus, S. albiflaviniger, S. albireticuli, S. albofaciens, S. alboflavus, S. albogriseolus, S. albolongus, S. alboniger, S. albospinus, S. albosporeus, S. albo. subsp. albosporeus, S. albo. subsp. labilomyceticus, S. alboverticillatus, S. albovinaceus, S. alboviridis, S. albulus, S. albus, S. albu. subsp. albus, S. albu. subsp. pathocidicus, S. aldersoniae, S. almquistii, S. alhi, S. althioticus, S. amakusaensis, S. ambofaciens, S. aminophilus, S. amritsarensis, S. anandii, S. angustmyceticus, S. anthocyanicus, S. antibioticus, S. antimycoticus, S. anulatus, S. aomiensis, S. arabicus, S. araujoniae, S. ardus, S. arenae, S. argenteolus, S. armeniacus, S. artemisiae, S. ascomycinicus, S. asiaticus, S. asterosporus, S. atacamensis, S. atratus, S. atriruber, S. atroaurantiacus, S. atroolivaceus, S. atrovirens, S. aurantiacus, S. aurantiogriseus, S. auratus, S. aureocirculatus, S. aureofaciens, S. aureorectus, S. aureoversilis, S. aureoverticillatus, S. aureus, S. avellaneus, S. avermectinius, S. avermitilis, S. avicenniae, S. avidinii, S. axinellae, S. azaticus, S. azureus, S. baarnensis, S. bacillaris, S. badius, S. baldaccii, S. baliensis, S. bambergiensis, S. bangladeshensis, S. barkulensis, S. beijiangensis, S. bellus, S. bikiniensis, S. biverticillatus, S. blastmyceticus, S. bluensis, S. bobili, S. bottropensis, S. brasiliensis, S. brevispora, S. bullii, S. bungoensis, S. burgazadensis, S. cacaoi, S. cac. subsp. asoensis, S. cac. subsp. cacaoi, S. caelestis, S. caeruleatus, S. caeruleus, S. calidiresistens, S. californicus, S. calvus, S. canarius, S. candidus, S. canescens, S. cangkringensis, S. caniferus, S. canus, S. capillispiralis, S. capoamus, S. carpaticus, S. carpinensis, S. castelarensis, S. catbensis, S. catenulae, S. caviscabies, S. cavourensis, S. cav. subsp. cavourensis, S. cav. subsp. washingtonensis, S. cellostaticus, S. celluloflavus, S. cellulolyticus, S. cellulosae, S. champavatii, S. chartreusis, S. chattanoogensis, S. cheonanensis, S. chiangmaiensis, S. chibaensis, S. chilikensis, S. chlorus, S. chrestomyceticus, S. chromofuscus, S. chryseus, S. chrysomallus, S. ch. subsp. chrysomallus, S. ch. subsp. fumigatus, S. chumphonensis, S. cinereorectus, S. cinereoruber, S. ci. subsp. cinereoruber, S. ci. subsp. fructofermentans, S. cinereospinus, S. cinereus, S. cinerochromogenes, S. cinnabarinus, S. cinnamonensis, S. cinnamoneus, S. cirratus, S. ciscaucasicus, S. citreofluorescens, S. clavifer, S. clavuligerus, S. coacervatus, S. cochleatus, S. cocklensis, S. coelescens, S. coelicoflavus, S. coelicolor, S. coeruleoflavus, S. coeruleofuscus, S. coeruleoprunus, S. coeruleorubidus, S. coerulescens, S. collinus, S. colombiensis, S. corchorusii, S. costaricanus, S. cremeus, S. crystallinus, S. curacoi, S. cuspidosporus, S. cyaneofuscatus, S. cyaneus, S. cyanoalbus, S. cystargineus, S. daghestanicus, S. daliensis, S. deccanensis, S. decoyicus, S. demainii, S. deserti, S. diastaticus, S. d. subsp. ardesiacus, S. d. subsp. diastaticus, S. diastatochromogenes, S. distallicus, S. djakartensis, S. drozdowiczii, S. durhamensis, S. durmitorensis, S. echinatus, S. echinoruber, S. ederensis, S. ehimensis, S. emeiensis, S. endophyticus, S. endus, S. enissocaesilis, S. erringtonii, S. erumpens, S. erythraeus, S. erythrogriseus, S. eurocidicus, S. europaeiscabiei, S. eurythermus, S. exfoliatus, S. felleus, S. fenghuangensis, S. ferralitis, S. fervens, S. filamentosus, S. fildesensis, S. filipinensis, S. fimbriatus, S. fimicarius, S. finlayi, S. flaveolus, S. flaveus, S. flavidofuscus, S. flavidovirens, S. flaviscleroticus, S. flavofungini, S. flavofuscus, S. flavogriseus, S. flavopersicus, S. flavotricini, S. flavovariabilis, S. flavovirens, S. flavoviridis, S. flocculus, S. floridae, S. fluorescens, S. fradiae, S. fragilis, S. fukangensis, S. fulvissimus, S. fulvorobeus, S. fumanus, S. fumigatiscleroticus, S. galbus, S. galilaeus, S. gancidicus, S. gardneri, S. gelaticus, S. geldanamycininus, S. geysiriensis, S. ghanaensis, S. gibsonii, S. glaucescens, S. glauciniger, S. glaucosporus, S. glaucus, S. globisporus, S. gl. subsp. caucasicus, S. gl. subsp. flavofuscus, S. gl. subsp. globisporus, S. globosus, S. glomeratus, S. glomeroaurantiacus, S. glycovorans, S. gobitricini, S. goshikiensis, S. gougerotii, S. graminearus, S. gramineus, S. graminifolii, S. graminilatus, S. graminisoli, S. graminofaciens, S. griseiniger, S. griseinus, S. griseoaurantiacus, S. griseobrunneus, S*. *griseocarneus, S. griseochromogenes, S. griseoflavus, S. griseofuscus, S. griseoincarnatus, S. griseoloalbus, S. griseolosporeus, S. griseolus, S. griseoluteus, S. griseomycini, S. griseoplanus, S. griseorubens, S. griseoruber, S. griseorubiginosus, S*. *griseosporeus, S. griseostramineus, S. griseoverticillatus, S. griseoviridis, S. griseus, S. gr. subsp. alpha, S. gr. subsp. cretosus, S. gr. subsp. griseus, S. gr. subsp. solvifaciens, S. guanduensis, S. gulbargensis, S. hachijoensis, S. hainanensis, S. haliclonae, S. halophytocola, S. halstedii, S. hawaiiensis, S. hebeiensis, S. heilongjiangensis, S. heliomycini, S. helvaticus, S. herbaceus, S. herbaricolor, S. himastatinicus,* S. *hiroshimensis, S. hirsutus, S. hokutonensis, S. hoynatensis, S. humidus, S. humiferus, S. hyderabadensis, S. hydrogenans, S. hygroscopicus, S. h. subsp. angustmyceticus, S. h. subsp. decoyicus, S. h. subsp. glebosus, S. h. subsp. hygroscopicus, S. h. subsp. ossamyceticus, S. hypolithicus, S. iakyrus, S. iconiensis, S. incanus, S. indiaensis, S. indicus, S. indigoferus, S. indonesiensis, S. intermedius, S. inusitatus, S. ipomoeae, S. iranensis, S. janthinus, S. javensis, S. jietaisiensis, S. jiujiangensis, S. kaempferi, S. kanamyceticus, S. karpasiensis, S. kashimirensis, S. kasugaensis, S. katrae, S. kentuckensis, S. kifunensis, S. kishiwadensis, S. koyangensis, S. kunmingensis, S. kurssanovii, S. labedae, S. laceyi, S. lacticiproducens, S. laculatispora, S. ladakanum, S. lanatus, S. lannensis, S. lateritius, S. laurentii, S. lavendofoliae, S. lavendulae, S. la. subsp. grasserius, S. la. subsp. lavendulae, S. lavenduligriseus, S. lavendulocolor, S. leeuwenhoekii, S. levis, S. libani, S. li. subsp. libani, S. li. subsp. rufus, S. lienomycini, S. lilacinus, S. limosus, S. lincolnensis, S. lipmanii, S. litmocidini, S. lividans, S. lomondensis, S. longisporoflavus, S. longispororuber, S. longisporus, S. longwoodensis, S. lopnurensis, S. lucensis, S. lunalinharesii, S. luridiscabiei, S. luridus, S. lusitanus, S. luteireticuli, S. luteogriseus, S. luteosporeus, S. luteoverticillatus, S. lydicus, S. macrosporus, S. malachitofuscus, S. malachitospinus, S. malaysiensis, S. marinus, S. marokkonensis, S*. *mashuensis, S. massasporeus, S. matensis, S. mauvecolor, S. mayteni, S. mediocidicus, S. mediolani, S. megasporus, S. melanogenes, S. melanosporofaciens, S. mexicanus, S. michiganensis, S. microflavus, S. milbemycinicus, S. minutiscleroticus, S. mirabilis, S. misakiensis, S. misionensis, S. mobaraensis, S. monomycini, S. mordarskii, S. morookaense, S. muensis, S. murinus, S. mutabilis, S. mutomycini, S. naganishii, S. nanhaiensis, S. nanshensis, S. narbonensis, S. nashvillensis, S. netropsis, S. neyagawaensis, S. niger, S. nigrescens, S. nigrifaciens, S. nitrosporeus, S. niveiscabiei, S. niveoruber, S. niveus, S. noboritoensis, S. nodosus, S. nogalater, S. nojiriensis, S. noursei, S. novaecaesareae, S. ochraceiscleroticus, S. odorifer, S. olivaceiscleroticus, S. olivaceoviridis, S. olivaceus, S. olivochromogenes, S. olivomycini, S. olivoreticuli, S. olivoverticillatus, S. olivoviridis, S. omiyaensis, S. orinoci, S. osmaniensis, S. pactum, S. panacagri, S. panaciradicis, S. paracochleatus, S. paradoxus, S. parvisporogenes, S. parvulus, S. parvus, S. pathocidini, S. paucisporeus, S. peucetius, S. phaeochromogenes, S. phaeofaciens, S. phaeogriseichromatogenes, S. phaeoluteichromatogenes, S. phaeoluteigriseus, S. phaeopurpureus, S. phaeoviridis, S. pharetrae, S. pharmamarensis, S. phosalacineus, S. phytohabitans, S. pilosus, S. platensis, S. plicatus, S. plumbiresistens, S. pluricolorescens, S. pluripotens, S. polyantibioticus, S. polychromogenes, S. poonensis, S. praecox, S. prasinopilosus, S. prasinosporus, S. prasinus, S. pratens, S. pratensis, S. prunicolor, S. psammoticus, S. pseudoechinosporeus, S. pseudogriseolus, S. pseudovenezuelae, S. pulveraceus, S. puniceus, S. puniciscabiei, S. purpeofuscus, S. purpurascens, S. purpureus, S. purpurogeneiscleroticus, S. qinglanensis, S. racemochromogenes, S. radiopugnans, S. rameus, S. ramulosus, S. rangoonensis, S. rapamycinicus, S. recifensis, S. rectiverticillatus, S. rectiviolaceus, S. regensis, S. resistomycificus, S. reticuliscabiei, S. rhizosphaericus, S. rhizophilus, S. rimosus, S. ri. subsp. paromomycinus, S. ri. subsp. rimosus, S. rishiriensis, S. rochei, S. rosealbus, S. roseiscleroticus, S. roseodiastaticus, S. roseoflavus, S. roseofulvus, S. roseolilacinus, S. roseolus, S. roseosporus, S. roseoverticillatus, S. roseoviolaceus, S. roseoviridis, S. ruanii, S. ruber, S. rubidus, S. rubiginosohelvolus, S. rubiginosus, S. rubrogriseus, S. rubrus, S. rutgersensis, S. ru. subsp. castelarensis, S. ru. subsp. rutgersensis, S. salmonis, S. sampsonii, S. samsunensis, S. sanglieri, S. sannanensis, S. sanyensis, S. sapporonensis, S. scabiei, S. scabrisporus, S. sclerotialus, S. scopiformis, S. scopuliridis, S. sedi, S. seoulensis, S. septatus, S. seranimatus, S. setae, S. setonii, S. shaanxiensis, S. shenzhenensis, S. showdoensis, S. silaceus, S. sindenensis, S. sioyaensis, S. smyrnaeus, S. sodiiphilus, S. somaliensis, S. sparsogenes, S. sparsus, S. specialis, S. spectabilis, S. speibonae, S. speleomycini, S. spheroides, S. spinoverrucosus, S. spiralis, S. spiroverticillatus, S. spitsbergensis, S. spongiae, S. sporocinereus, S. sporoclivatus, S. spororaveus, S. sporoverrucosus, S. staurosporininus, S. stelliscabiei, S. stramineus, S. subrutilus, S. sulfonofaciens, S. sulphureus, S. sundarbansensis, S. synnematoformans, S. syringium, S. tacrolimicus, S. tanashiensis, S. tateyamensis, S. tauricus, S. tendae, S. termitum, S. thermoalcalitolerans, S. thermoautotrophicus, S. thermocarboxydovorans, S. thermocarboxydus, S. thermocoprophilus, S. thermodiastaticus, S. thermogriseus, S. thermolineatus, S. thermonitrificans, S. thermospinosisporus, S. thermoviolaceus, S. t. subsp. apingens, S. t. subsp. thermoviolaceus, S. thermovulgaris, S. thinghirensis, S. thioluteus, S. torulosus, S. toxytricini, S. tricolor, S. tritolerans, S. tubercidicus, S. tuirus, S. tunisiensis, S. turgidiscabies, S. umbrinus, S. variabilis, S. variegatus, S. varsoviensis, S. vastus, S. venezuelae, S. vietnamensis, S. vinaceus, S. vinaceusdrappus, S. violaceochromogenes, S. violaceolatus, S. violaceorectus, S. violaceoruber, S. violaceorubidus, S. violaceus, S. violaceusniger, S. violarus, S. violascens, S. violatus, S. violens, S. virens, S. virginiae, S. viridiflavus, S. viridiviolaceus, S. viridis, S. viridobrunneus, S. viridochromogenes, S. viridodiastaticus, S. viridosporus, S. vitaminophilus, S. wedmorensis, S. wellingtoniae, S. werraensis, S. willmorei, S. wuyuanensis, S. xanthochromogenes, S. xanthocidicus, S. xantholiticus, S. xanthophaeus, S. xiamenensis, S. xinghaiensis, S. xishensis, S. yaanensis, S. yanglinensis, S. yanii, S. yatensis, S. yeochonensis, S. yerevanensis, S. yogyakartensis, S. yokosukanensis, S. youssoufiensis, S. yunnanensis, S. zaomyceticus, S. zhaozhouensis, S. zinciresistens,* and *S. ziwulingensis.* The actinomycete of the genus Streptomyces may be, for example, an antibiotic-producing bacterium. Examples of the antibiotic include streptomycin, kanamycin, rapamycin, daunomycin, aclacinomycin, tetracycline, erythromycin, rifampicin, bleomycin, and vancomycin.

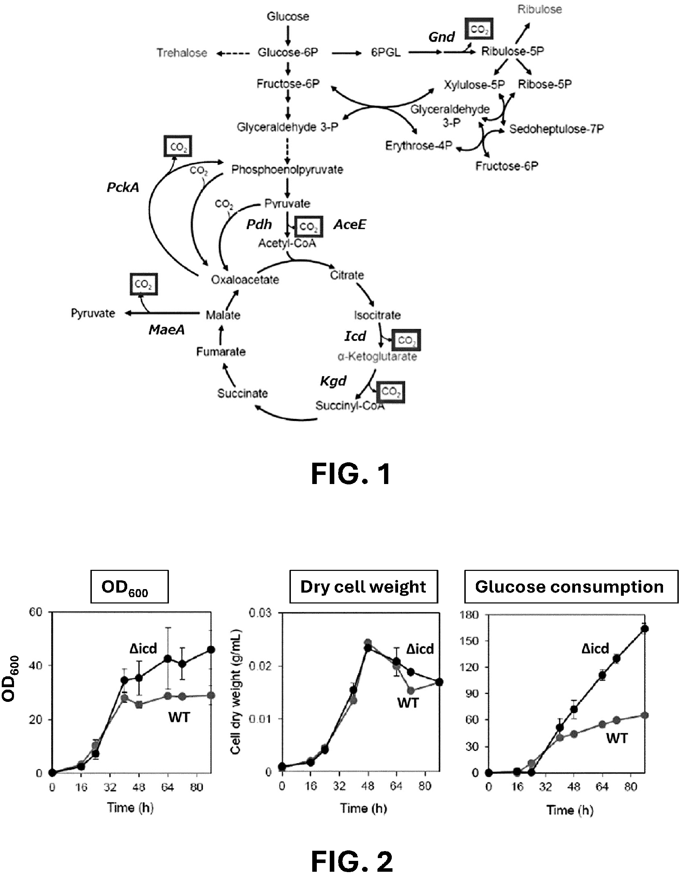

In the present specification, "aerobic conditions" mean conditions under which aerobes perform aerobic respiration. Aerobic respiration is carried out by a metabolic system including glycolysis, the citric acid cycle, and the electron transport chain, and thereby usually, saccharides consume oxygen to produce carbon dioxide, water, and ATP, which is energy. Glycolysis is a pathway that produces pyruvate from glucose via glucose-6-phosphate, as shown in FIG. 1. Pyruvate produced in glycolysis is converted to acetyl-CoA by the pyruvate dehydrogenase complex and introduced into the citric acid cycle (for convenience, in the present specification, the pathway up to producing acetyl-CoA is classified into glycolysis). Acetyl-CoA is subsequently metabolized into the citric acid cycle. The citric acid cycle is a cycle also called the TCA cycle. The citric acid cycle produces NADH, FADH₂, GTP, and the like. The electron transport chain oxidizes NADH and FADH₂ to produce ATP. By cultivating aerobes under aerobic conditions, ATP is produced from saccharides in the metabolic system including glycolysis, the citric acid cycle, and the electron transport chain. In contrast, "anaerobic conditions" are conditions under which anaerobic organisms perform energy production and growth without using oxygen. A person skilled in the art can appropriately cultivate aerobes under aerobic conditions.

Bacterial cultivation is composed of a lag phase, a logarithmic growth phase, and a stationary phase. For a certain period after the start of cell culture, bacteria do not divide, and the cell number does not change. This period is called a lag phase. It is considered that bacteria perform cell repair, adjust metabolism, accumulate carbonic acid, etc., during the lag phase. Thereafter, the bacteria start dividing, begin to grow slowly, gradually increase the growth rate, and the generation time becomes constant so that the bacterial cells grow logarithmically. This period is called a logarithmic growth phase. When cultivation is continued and cell density increases, the period in which bacterial cells grow logarithmically (logarithmic growth phase) ends. Typically, the rate of cell division of bacterial cells gradually decreases and the viable cell count becomes constant, but depending on the bacterial species, bacterial cells may continue to grow slowly. This period is generally called a stationary phase. Substance production using bacteria is mainly performed from the logarithmic growth phase to the stationary phase. Since the boundary between a logarithmic growth phase and a stationary phase is generally not clear, in the present specification, hereinafter, a logarithmic growth phase is defined as a period in which the growth of bacterial cells becomes active after the start of cultivation and the doubling time can be regarded as constant, and a stationary phase is defined as a period in which the doubling time of bacterial cells is clearly extended and the viable cell count can be regarded as constant, or in the case of a bacterial species that continues to grow slowly, a period in which the growth of bacterial cells is stable with a doubling time significantly extended compared to that in the logarithmic growth phase (for example, 2 times or more, 3 times or more, 4 times or more, 5 times or more, or 10 times or more of that in the logarithmic growth phase, differing depending on the bacterial species).

In the present specification, "yield relative to carbon" means an amount incorporated as carbon of a specific substance among carbon contained in a carbon source (e.g., glucose) contained in nutrients.

In the present specification, "carbon source" means an organic substance that provides an energy source and bacterial cell carbon components in heterotrophic cells such as aerobes. In an embodiment, as the carbon source, for example, but not particularly limited to, sugars, for example monosaccharides, particularly glucose can be preferably used.

### <Bacterium and Selection Method of the Present Disclosure>

According to the examples described later, there is a large difference between bacteria in the yield of CO₂ relative to carbon. In particular, there are bacteria whose yield of CO₂ relative to carbon exceeds about 50% in at least a logarithmic growth phase or a stationary phase (preferably in a logarithmic growth phase). The present disclosure provides such a bacterium, and a method for selecting, obtaining, or isolating such a bacterium.

According to the present disclosure, provided is a bacterium whose yield of CO₂ relative to carbon per glucose exceeds, for example, about 50% in at least a logarithmic growth phase or a stationary phase. The bacterium can metabolize glucose to produce energy. In an embodiment, provided is a bacterium whose yield of CO₂ relative to carbon per glucose exceeds about 60%, exceeds about 70%, exceeds about 80%, exceeds about 90%, or exceeds about 95% in at least a logarithmic growth phase or a stationary phase. In a preferred embodiment, the bacterium may be an actinomycete of the genus Streptomyces. In a specific embodiment, the actinomycete may be *Streptomyces lividans.* According to the present disclosure, a metabolic enzyme gene-disrupted strain of these bacteria (e.g., actinomycetes) is provided. A bacterium having a high yield of CO₂ relative to carbon per glucose is useful as a subject to undergo modification of the present disclosure. In an embodiment, the yield of CO₂ relative to carbon per glucose is higher than that of *E. coli* in at least a logarithmic growth phase or a stationary phase (e.g., about 1.1 times or more, about 1.2 times or more, about 1.3 times or more, about 1.4 times or more, or about 1.5 times or more). Alternatively, the yield of CO₂ relative to carbon per glucose is higher than that of *Corynebacterium glutamicum,* which is a coryneform bacterium, in at least a logarithmic growth phase or a stationary phase (e.g., about 1.1 times or more, about 1.2 times or more, about 1.3 times or more, about 1.4 times or more, or about 1.5 times or more). In a preferred embodiment, the yield relative to carbon is of a logarithmic growth phase.

According to the present disclosure, provided is a method for selecting, obtaining, or isolating the bacterium. The method of the present disclosure includes providing an isolated bacterium. Isolation may preferably be cloning. The method of the present disclosure may further include measuring a yield relative to carbon of carbon dioxide of the bacterium in a logarithmic growth phase or a stationary phase in aerobic culture in a medium containing a sufficient amount of a carbon source and other nutrients (e.g., for actinomycetes, 1.8 g/L TSB medium, containing 50 g/L glucose, pH 7.0). The yield relative to carbon can be calculated from the amount of glucose consumed and the amount of metabolite produced. The amount of glucose and the amount of metabolite can be measured by a conventional method. Also, the carbon dioxide emission amount can be measured by a conventional method using an exhaust gas analyzer (e.g., OFF-GAS Jr. manufactured by Biott Corporation) and a mass spectrometer or the like. The amount of glucose and the amount of metabolite can be measured by, for example, but not particularly limited to, separation of components by high performance liquid chromatography and subsequent component analysis. Component analysis can be performed by various techniques such as mass spectrometry, a differential refractive index detector, and a UV detector alone or in combination of pluralities thereof.

Based on the measurement result of the yield relative to carbon of carbon dioxide, a bacterium whose yield relative to carbon exceeds, for example, about 50%, exceeds about 60%, exceeds about 70%, exceeds about 80%, exceeds about 90%, or exceeds about 95% can be selected, obtained, or isolated. Thus, a bacterium having a high yield relative to carbon of carbon dioxide can be obtained. In this method, the bacterium can be a bacterial cell isolated from nature (soil, a living body, a biological sample, water, etc.). In this method, the bacterium may be a bacterium established as a strain. In this method, the bacterium may be preferably an actinomycete of the genus Streptomyces. The obtained bacterium can be subjected to cloning. Cloning can be achieved, for example, by seeding on a solid medium and picking up a single colony. Regarding actinomycetes, for example, the actinomycete may be cloned by seeding spores or protoplasted cells on a solid medium and picking up a single colony. Thus, a person skilled in the art can clone the bacterium by an appropriately suitable method.

### <Metabolic Enzyme Gene-Disrupted Strain of Bacterium of the Present Disclosure and Method for Producing the Same>

According to the present disclosure, provided is a method for producing a metabolic enzyme gene-disrupted strain of a bacterium.

The bacterium may be an aerobe. In an embodiment, the bacterium may be an actinomycete of the genus Streptomyces.

In a preferred embodiment, the bacterium may be a bacterium obtained by the above method. That is, the bacterium may be a bacterium whose yield of CO₂ relative to carbon per glucose exceeds, for example, about 50% in at least a logarithmic growth phase or a stationary phase, and, may more preferably be a bacterium exceeding about 60%, exceeding about 70%, exceeding about 80%, exceeding about 90%, or exceeding about 95% for the yield relative to carbon in at least a logarithmic growth phase or a stationary phase. The bacterium may be an aerobe, and preferably may be an actinomycete of the genus Streptomyces.

The method for producing a metabolic enzyme gene-disrupted strain of a bacterium includes providing the above bacterium.

In the method for producing a metabolic enzyme gene-disrupted strain of a bacterium, the bacterium to be modified is isolated, and preferably cloned.

It is beneficial to reduce the yield of CO₂ relative to carbon per glucose of the bacterium. Metabolic reactions that generate CO₂ in metabolic pathways are known. For the purpose of reducing the yield of CO₂ relative to carbon, one or two or more enzymes catalyzing such a metabolic reaction can be disrupted by genetic engineering.

The method for producing a metabolic enzyme gene-disrupted strain of a bacterium includes disrupting an enzyme catalyzing a metabolic reaction generating CO₂ in the metabolic pathway of the bacterium. Disruption is achieved by one or more modifications selected from the group consisting of inhibition and loss of expression, inhibition and loss of function, and deletion of a gene. Disruption reduces the metabolic activity of the enzyme to, for example, about half or less, about 30% or less, about 20% or less, about 10% or less, or about 5% or less, or to a detection limit or less, although not particularly limited as long as the yield of carbon dioxide relative to sugar decreases. When a certain metabolic reaction is carried out by a plurality of enzymes, disruption is performed on a gene encoding at least one of the enzymes (e.g., genes encoding all enzymes). According to the present disclosure, provided is a metabolic enzyme gene-disrupted strain in which one or more, or two or more of enzymes catalyzing a metabolic reaction generating CO₂ in the metabolic pathway of the bacterium are disrupted.

Hereinafter, enzymes responsible for a metabolic reaction producing CO₂ are exemplified, but are not limited thereto. Also, orthologs of the following enzymes having an equivalent function can be preferably disrupted as enzymes responsible for a metabolic reaction producing CO₂.

Examples of the enzyme responsible for a metabolic reaction producing CO₂ in a metabolic pathway include, but are not particularly limited to, isocitrate dehydrogenase (EC 1.1.1.41). Isocitrate dehydrogenase (EC 1.1.1.41) is an enzyme that catalyzes a reaction producing CO₂ and α-ketoglutarate from isocitrate. Examples of the gene encoding such an enzyme include icd, SLI_7202, and the like.

Examples of the enzyme responsible for a metabolic reaction producing CO₂ in a metabolic pathway include, but are not particularly limited to, pyruvate decarboxylase (EC 4.1.1.1). Pyruvate decarboxylase (EC 4.1.1.1) is an enzyme that catalyzes a reaction producing CO₂ and acetaldehyde from pyruvate.

Examples of the enzyme responsible for a metabolic reaction producing CO₂ in a metabolic pathway include, but are not particularly limited to, phosphogluconate dehydrogenase (EC 1.1.1.44, 1.1.1.343, or 1.1.1.351). Phosphogluconate dehydrogenase (EC 1.1.1.44, 1.1.1.343, or 1.1.1.351) is an enzyme that catalyzes a production reaction of ribulose-5-phosphate, NADPH₂⁺, and CO₂ from 6-phosphogluconate and NADP⁺ in the pentose phosphate pathway. Examples of the gene encoding such an enzyme include gnd, SLI_4134, SLI_7003, SLI_1209, etc., and combinations thereof.

Examples of the enzyme responsible for a metabolic reaction producing CO₂ in a metabolic pathway include, but are not particularly limited to, pyruvate dehydrogenase (EC 1.2.4.1), dihydrolipoyl transacetylase (EC 2.3.1.12), and dihydrolipoyl dehydrogenase (EC 1.8.1.4). Pyruvate dehydrogenase (EC 1.2.4.1), dihydrolipoyl transacetylase (EC 2.3.1.12), and dihydrolipoyl dehydrogenase (EC 1.8.1.4) may be enzymes that form a pyruvate dehydrogenase complex and produce acetyl-CoA, NADH, and CO₂ from pyruvate, CoA-SH, and NAD⁺. Examples of the gene encoding such an enzyme include aceE, SLI_2705, SLI_2510, SLI_7339, etc., and combinations thereof.

Examples of the enzyme responsible for a metabolic reaction producing CO₂ in a metabolic pathway include, but are not particularly limited to, α-ketoglutarate dehydrogenase or oxoglutarate dehydrogenase (EC 1.2.4.2, EC 1.2.7.3, or EC 1.2.7.11), dihydrolipoamide S-succinyltransferase (EC 2.3.1.61), and dihydrolipoyl dehydrogenase (EC 1.8.1.4). Oxoglutarate dehydrogenase (EC 1.2.4.2, EC 1.2.7.3, or EC 1.2.7.11), dihydrolipoamide S-succinyltransferase (EC 2.3.1.61), and dihydrolipoyl dehydrogenase (EC 1.8.1.4) form an oxoglutarate dehydrogenase complex and produce succinyl-CoA, NADH, H⁺, and CO₂ from α-ketoglutarate, NAD⁺, and CoA-SH. Examples of the gene encoding such an enzyme include kgd, SLI_2508, SLI_5576, etc., and combinations thereof.

Examples of the enzyme responsible for a metabolic reaction producing CO₂ in a metabolic pathway include, but are not particularly limited to, malate dehydrogenase (EC 1.1.1.38, EC 1.1.1.39, or EC 1.1.1.40). Malate dehydrogenase (EC 1.1.1.38, EC 1.1.1.39, or EC 1.1.1.40) produces pyruvate, CO₂, and NADH from malate and NAD⁺. Examples of the gene encoding such an enzyme include maeA, SLI_5552, SLI_3297, etc., and combinations thereof.

Examples of the enzyme responsible for a metabolic reaction producing CO₂ in a metabolic pathway include, but are not particularly limited to, phosphoenolpyruvate carboxykinase (EC 4.1.1.32, EC 4.1.1.38, EC 4.1.1.49). Phosphoenolpyruvate carboxykinase (EC 4.1.1.32, EC 4.1.1.38, EC 4.1.1.49) all produce phosphoenolpyruvate and CO₂ from oxaloacetate. Examples of such an enzyme gene include, for example, pckA, SLI_5252, and the like.

Examples of the enzyme responsible for a metabolic reaction producing CO₂ in a metabolic pathway include, but are not particularly limited to, glutamate decarboxylase, hydroxypyruvate decarboxylase, 3-oxoacyl synthase, and aspartate decarboxylase. Metabolic reactions of cells have been elucidated in detail, and a person skilled in the art will be able to appropriately identify an enzyme responsible for a metabolic reaction producing CO₂ and appropriately disrupt the gene thereof.

In the method of the present disclosure, 1 or more, 2 or more, 3 or more, 4 or more, or 5 or more of enzymes responsible for a metabolic reaction producing CO₂ can be disrupted. Thereby, it can be expected that the yield relative to carbon of carbon dioxide from glucose in the disrupted strain decreases.

According to the present disclosure, provided is a bacterium (metabolic enzyme gene-disrupted strain) in which 1 or more, 2 or more, 3 or more, 4 or more, or 5 or more of enzymes responsible for a metabolic reaction producing CO₂ are disrupted. In an embodiment, the bacterium is isolated. In an embodiment, the bacterium is present in a culture. In an embodiment, the bacterium is cloned. In an embodiment, the bacterium to be modified may be an aerobe. In an embodiment, the bacterium may be an actinomycete of the genus Streptomyces. In a preferred embodiment, the bacterium to be modified may be a bacterium (preferably an actinomycete, e.g., an actinomycete of the genus Streptomyces) whose yield of CO₂ relative to carbon per glucose exceeds about 50% in at least a logarithmic growth phase or a stationary phase, and more preferably, the bacterium may be a bacterium exceeding about 60%, exceeding about 70%, exceeding about 80%, exceeding about 90%, or exceeding about 95% in at least a logarithmic growth phase or a stationary phase. In a preferred embodiment, the bacterium may be *Streptomyces lividans* (e.g., strain 1326).

The yield of CO₂ relative to carbon per glucose of the metabolic enzyme gene-disrupted strain may be at most about 90% or less, preferably about 80% or less, more preferably about 70% or less, further preferably about 60% or less, and still more preferably about 50% or less compared to the strain before disruption of the metabolic enzyme gene in at least a logarithmic growth phase or a stationary phase. The yield of CO₂ relative to carbon per glucose of the metabolic enzyme gene-disrupted strain may be, for example, about 5% or more, about 10% or more, about 20% or more, about 30% or more, about 40% or more, or about 50% or more compared to the strain before metabolic modification in at least a logarithmic growth phase or a stationary phase. The yield of CO₂ relative to carbon per glucose of the metabolic enzyme gene-disrupted strain may be, for example, about 5% to about 90%, about 10% to about 90%, about 20% to about 90%, about 30% to about 90%, about 35% to about 80%, about 40% to about 70%, about 45% to about 60%, or about 50% compared to the strain before disruption of the metabolic enzyme gene in at least a logarithmic growth phase or a stationary phase.

In a preferred embodiment, the metabolic enzyme gene-disrupted strain maintains a growth ability of about 50% or more, about 60% or more, about 70% or more, about 80% or more, or about 90% or more compared to the strain before disruption of the metabolic enzyme gene. The growth ability can be determined, for example, by the dry cell weight in 1 mL of culture fluid after cultivation (e.g., 40 hours after the start of main culture).

### <Method of Cultivating Bacterium of the Present Disclosure>

According to the present disclosure, provided is a method for cultivating the gene-modified bacterium of the present disclosure. The method of the present disclosure includes preparing a gene-modified bacterium and cultivating the gene-modified bacterium in an environment suitable for cultivation. In a preferred embodiment, the environment suitable for cultivation may be conditions suitable for cultivation of a corresponding wild-type bacterium. In an embodiment, the environment suitable for cultivation may be an environment suitable for cultivation of the gene-modified bacterium. In an embodiment, the bacterium is cultivated under anaerobic conditions. In a preferred embodiment, the bacterium is cultivated under aerobic conditions, for example, while shaking or while aerating and stirring.

As the medium, a solid medium may be used, but a liquid medium is preferably used. As the medium, a medium containing components necessary for survival and/or substance production (metabolite production) of the bacterium, such as a carbon source and other nutrient sources (e.g., nitrogen source, inorganic salts, etc.) can be used. A person skilled in the art can appropriately prepare such a medium. The medium may be a synthetic medium or a semi-synthetic medium. The medium may contain basal media such as TSB medium, LB medium, YT medium (or 2xYT medium), NZY medium, M9 medium, SOC medium, and YPD medium; buffers; salts; pH adjusters; alkali metal ions such as potassium ions and sodium ions; alkaline earth metal ions such as calcium ions; and metal ions such as magnesium ions, iron ions, tin ions, cobalt ions, manganese ions, and zinc ions; phosphorus, and sulfur. The medium may contain, as a nitrogen source, a nitrogen source selected from the group consisting of ammonium salts, nitrates, amino acids, peptone, casein, casamino acid, urea, yeast extract, protein hydrolysate, yeast autolysate, casein pancreatic digest, soybean papain digest, tryptone, phytone peptone, meat extract, meat peptone, and tryptose. Examples of the carbon source include glucose, dextran, starch, beef extract, pyruvate, acetic acid, and the like. Examples of the inorganic salts include sodium salts such as sodium phosphate, sodium chloride, and sodium molybdate; potassium salts such as potassium phosphate, potassium iodide, and potassium chloride; calcium salts such as calcium chloride and calcium phosphate; magnesium salts such as magnesium chloride and magnesium sulfate; transition metal salts such as copper sulfate, manganese sulfate, zinc sulfate, and iron chloride; borates, and the like. The pH of the medium is not particularly limited but may be, for example, about 7, for example, about 6.5 to 7.5. The cultivation temperature differs depending on the bacterium, but may be, for example, 25°C to 37°C, for example, 25°C to 30°C. The cultivation period is not particularly limited but may be, for example, 1 day to 2 months, for example, 1 day to 1 month, for example, 1 day to 2 weeks, for example, 1 day to 1 week. A carbon source or other nutrient sources may be added to the medium during cultivation. A person skilled in the art can appropriately select cultivation conditions according to the bacterium.

In the method of the present disclosure, the cultivation includes cultivation of the bacterium or metabolic enzyme gene-disrupted strain in a logarithmic growth phase and a stationary phase. In the stationary phase, the bacterium or metabolic enzyme gene-disrupted strain can produce various metabolites. In particular, since the enzyme synthesizing carbon dioxide is disrupted in the metabolic enzyme gene-disrupted strain, the yield relative to carbon of the metabolite is improved, and the carbon source can be utilized for the production of the various metabolites. Examples of the metabolite include, but are not particularly limited to, undecylprodigiosin (Udp). Examples of the metabolite include metabolites of a metabolic pathway upstream or downstream of an enzymatic reaction accompanied by emission of CO₂, or metabolites produced utilizing the metabolites. In order to obtain a metabolite of a metabolic pathway downstream of an enzymatic reaction accompanied by emission of CO₂ or a metabolite produced utilizing the metabolite, it is conceivable to introduce, into cells, an intermediate of the metabolic pathway downstream of the enzymatic reaction accompanied by emission of CO₂. Introduction of the intermediate into cells can be done by addition of the intermediate to the medium. The intermediate can be used as a raw material of the metabolite in the cells.

According to an embodiment, by the modification, the yield of CO₂ relative to carbon can be reduced compared to unmodified cells, which can contribute to reduction of CO₂ production amount by cultivation. According to a preferred embodiment, by the modification, carbon that should have been consumed by the production of CO₂ can be used for substance production, and in this case, by the modification, substance production can be promoted compared to unmodified cells. According to a preferred embodiment, by the modification, proliferation of cells is not inhibited by 50% or more, 40% or more, 33% or more, 30% or more, 25% or more, 20% or more, 15% or more, or 10% or more. According to an embodiment, by the modification, glucose consumption is not decreased by 50% or more, 40% or more, 33% or more, 30% or more, 25% or more, 20% or more, 15% or more, or 10% or more compared to unmodified cells, or preferably, glucose consumption is not decreased or is increased. According to a preferred embodiment, while the yield of CO₂ relative to carbon is reduced compared to unmodified cells by the modification, proliferation of cells is not inhibited by 20% or more (preferably 10% or more or not inhibited at all) by the modification, and/or glucose consumption is not decreased or is increased. According to a preferred embodiment, while the yield of CO₂ relative to carbon is reduced compared to unmodified cells by the modification, proliferation of cells is not inhibited by 20% or more (preferably 10% or more or not inhibited at all) by the modification, and glucose consumption is not decreased or is increased.

### EXAMPLES

### Example 1: Yield relative to carbon of bacteria

*Escherichia coli,* a coryneform bacterium (*Corynebacterium glutamicum*), and an actinomycete (*Streptomyces lividans,* strain 1326) were prepared. As a culture medium, a synthetic medium was used. The synthetic medium contained 1.8 g/L TSB medium (pH 7.0) as a basal medium. The pH of the synthetic medium was adjusted with NaOH. The synthetic medium also contained an initial glucose concentration of 50 g/L. Each bacterium was cultured with shaking in 50 mL of the synthetic medium under conditions of 28°C and 160 rpm for 48 hours. Thereafter, jar fermenter cultivation was performed. Bacteria obtained by pre-culture were added into 1 L of the synthetic medium so as to have an optical density of 0.2. Glucose was further added so as to be 50 g/L 40 hours after cultivation. An appropriate amount of antifoaming agent was added to the medium. During cultivation, the amount of CO₂ in exhaust gas was measured using an exhaust gas analyzer (OFF-GAS Jr. manufactured by Biott Corporation).

Escherichia coli was recovered after 10 hours (logarithmic growth phase), the coryneform bacterium was recovered after 10 hours (logarithmic growth phase), and the actinomycete was recovered after 40 hours (logarithmic growth phase) and after 88 hours (stationary phase).

The medium containing the recovered bacteria was subjected to HPLC analysis, and the yield relative to carbon was measured. The HPLC analysis conditions were as follows.

| | |
|---|---|
| Column: | Aminex HPX-87H |
| Eluent: | 5 mM H₂SO₄ |
| Flow rate: | 0.6 mL/min |
| Temperature: | 50°C |
| Detector: | UV detector, and differential refractive index detector (RID) |

The results were as shown in Table 1.

**[Table 1]**

| Table 1: Yield relative to carbon in logarithmic growth phase of each bacterium | | | |
|---|---|---|---|
| | *Escherichia coli* | *Coryneform bacterium* | *Actinomycete* |
| CO₂ | 49. 7% | 46.4% | 69. 2% |
| Bacterial cells | 39. 7% | 47.6% | 29. 0% |
| Others | 10. 7% | 6.0% | 7.5% |

As shown in Table 1, it was revealed that the examined actinomycete strain had a high yield of CO₂ relative to carbon in the logarithmic growth phase and converted most of the glucose into CO₂. Although data are not shown in Table 1, the yield of CO₂ relative to carbon in the stationary phase of the actinomycete showed a higher value than in the logarithmic growth phase. From this, it was revealed that there are species and strains having a high yield of CO₂ relative to carbon among bacteria (particularly actinomycetes).

The high yield of CO₂ relative to carbon is an indicator of how much carbon derived from glucose is used for the production of CO₂. When bacteria are utilized for substance production, the high yield of CO₂ relative to carbon can have a negative impact on substance production. This is because carbon derived from glucose is consumed for the production of CO₂, which can inhibit utilization for substance production.

### Example 2: Preparation of enzyme-disrupted strains and yield of CO₂ relative to carbon

### Example 2-1: Yield relative to carbon and glucose consumption amount in Δicd

Enzymatic reactions producing CO₂ exist in metabolic pathways such as glycolysis, TCA cycle, and pentose phosphate pathway (see FIG. 1). A deletion strain (Δicd) of an enzyme (icd) producing α-ketoglutarate and CO₂ from isocitrate was prepared, and the bacterial cell amount, glucose consumption amount, and yield of CO₂ relative to carbon during cultivation were measured. Cultivation was performed under the same conditions as the wild-type strain (WT) except that the initial glucose concentration and the added glucose concentration were 100 g/L, and bacterial cells were recovered. Measurement of ICD activity in the icd-disrupted strain was performed by measuring the increase in NADPH produced when isocitrate is converted to 2-ketoglutarate by ICD by measuring absorbance at 340 nm. As a result, ICD activity was significantly reduced in the Δicd strain.

**[Table 2]**

| Table 2: Yield relative to carbon ratio in stationary phase of Δicd strain | | |
|---|---|---|
| | Parent strain | Δicd |
| CO yield relative to carbon ratio | 1.0 | 0.5 |

As shown in Table 2, in the stationary phase, the yield of CO₂ relative to carbon in the Δicd strain decreased to about half compared to the wild-type strain (WT).

From the analysis of yield relative to carbon, it was also found that in the logarithmic growth phase of the Δicd strain, the yields relative to carbon of pyruvate, isocitrate or citrate, and ribulose increased. Also, in the stationary phase of the Δicd strain, the yields relative to carbon of ribulose and pyruvate increased.

From the analysis of yield relative to carbon, furthermore, in the stationary phase of the Δicd strain, in addition to the dramatic decrease in the yield of CO₂ relative to carbon, yields relative to carbon of a plurality of further unidentified metabolites accounted for 40.7% in total, from which it could be understood that carbon utilization efficiency in bacterial cells was greatly improved. Thus, in the Δicd strain, disruption of the enzyme responsible for the enzymatic reaction producing CO₂ caused a dramatic change in the yield of CO₂ relative to carbon.

This means that in a bacterium where utilization of a carbon source is strongly constrained by emission of carbon dioxide, the constraint can be released, and a state where the carbon source can be more effectively used in metabolic reactions can be induced. And, in this example, the effectiveness of gene disruption of the enzyme responsible for a metabolic reaction producing carbon dioxide was demonstrated.

Also, as shown in FIG. 2, a 2.6-fold increase in glucose consumption amount was recognized in the Δicd strain compared to the wild-type strain (WT).

### Example 2-2: Yield relative to carbon and glucose consumption amount in ΔpckA, ΔmaeA, and Δgnd

As enzyme genes producing CO₂ other than the icd-disrupted strain, a deficient strain (ΔpckA) of an enzyme (pckA) producing phosphoenolpyruvate from oxaloacetate, a deficient strain (ΔmaeA) of an enzyme (maeA) producing pyruvate from malate, and a deficient strain (Δgnd) of an enzyme (gnd) producing ribulose-5-phosphate from 6-phosphogluconate were prepared. Since there are two types of homologues for maeA, a double deficient strain was prepared, and for gnd, there are three types of homologues, but a deficient strain of only one type among them was prepared (see Table 9). The prepared disrupted strains and the wild-type strain were subjected to aerobic jar fermenter cultivation, and the bacterial cell amount, glucose consumption amount, and yield of CO₂ relative to carbon during cultivation were measured. Cultivation was performed under the same culture conditions as the wild-type strain.

**[Table 3]**

| Table 3: CO₂ yield relative to carbon ratio of each gene-disrupted strain relative to parent strain | | | | |
|---|---|---|---|---|
| | Parent strain | ΔpckA | ΔmaeA | Δgnd |
| CO₂ yield relative to carbon ratio | 1.0 | 0.86 | 0.88 | 0.86 |

As shown in Table 3, throughout the cultivation, the yield of CO₂ relative to carbon of each gene-disrupted strain decreased to 0.86 times in the ΔpckA strain, 0.88 times in the ΔmaeA strain, and 0.86 times in Δgnd, compared to that of the wild-type strain (parent strain), respectively. Also, the yield relative to carbon of CO₂ of each disrupted strain in the logarithmic growth phase was 0.82 times in the ΔpckA strain, 0.86 times in the ΔmaeA strain, and 0.92 times in Δgnd compared to that of the wild-type strain, and in the stationary phase, it was 0.97 times in the ΔpckA strain, 0.90 times in the ΔmaeA strain, and 0.81 times in Δgnd, respectively.

**[Table 4]**

| Table 4: Ratio of glucose consumption amount per unit bacterial cell of parent strain and each gene-disrupted strain | | | | |
|---|---|---|---|---|
| | Parent strain | ΔpckA | ΔmaeA | Δgnd |
| g glucose/g DCW | 1.0 | 1.3 | 1.9 | 1.2 |

As shown in Table 4, the glucose consumption amount per unit bacterial cell of each gene-disrupted strain increased to 1.3 times in the ΔpckA strain, 1.9 times in the ΔmaeA strain, and 1.2 times in Δgnd, respectively, compared to that of the wild-type strain (parent strain).

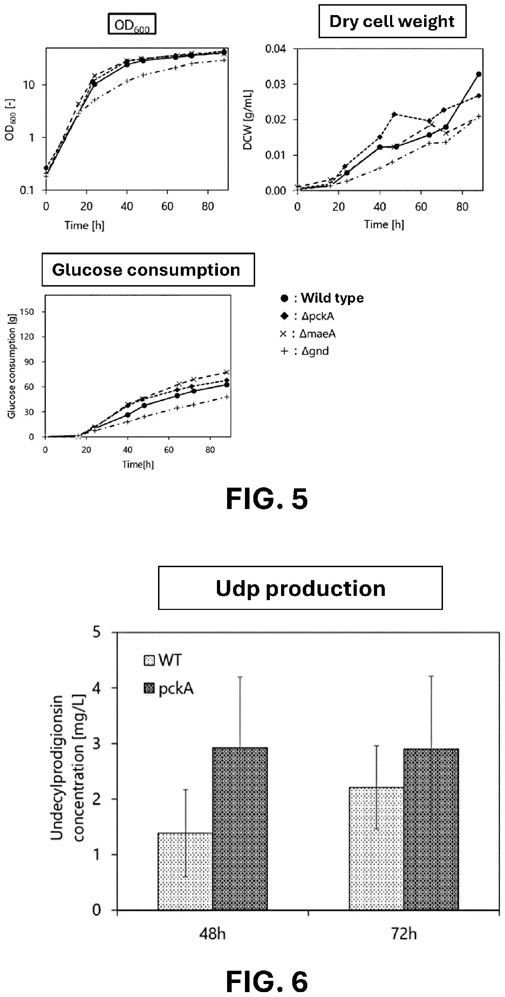

Also, as shown in FIG. 5, the yield of CO₂ relative to carbon of each gene-disrupted strain decreased compared to the wild-type strain, but bacterial cell growth did not increase compared to the wild-type strain, and furthermore, since the glucose consumption amount per bacterial cell increased, it can be expected that the decreased CO₂ carbon is used for metabolites.

### Example 3: Secondary metabolite production by enzyme gene-disrupted strain

### Example 3-1: Undecylprodigiosin (Udp) production by Δicd strain

The wild-type strain and the Δicd strain were each cultivated, and the production amount of undecylprodigiosin (Udp) was confirmed. Each strain was cultivated on a solid TSB medium (30°C, 48 hours), and pre-cultured in 10 mL of R2YE medium (see Table 5 and Table 6) while shaking (28°C, 160 rpm, 48 hours). The whole amount was added to 50 mL of R2YE medium, and main culture (28°C, 160 rpm, 72 hours) was performed. 10 mL of culture was recovered 48 hours and 72 hours after the start of main culture, respectively, and centrifuged to recover bacterial cells. 10 mL of methanol was added to treat the bacterial cells with methanol, and Udp was extracted. The absorbance (530 nm) of the supernatant containing extracted Udp was measured, and the Udp concentration was calculated.

**[Table 5]**

| Table 5: Composition of R2YE medium (1 L) | |
|---|---|
| Compound Name | Amount Used |
| Potassium sulfate | 0.25 g |
| Magnesium chloride hexahydrate | 10.1 g |
| Calcium chloride dihydrate | 2.95 g |
| D-(+)-glucose | 10 g |
| L(-)-proline | 3.0 g |
| Casamino acid | 0.1 g |
| TES | 5.75 g |
| Yeast extract | 5.0 g |
| Sucrose | 101.5 g |
| 1 N Sodium hydroxide | 11.5 mL |
| 0.5% Potassium dihydrogen phosphate | 10.0 mL |
| Trace element solution (B) | 2.0 mL |
| Water | Up to 1L |

**[Table 6]**

| Table 6: Composition of trace element solution (B) | |
|---|---|
| Compound Name | Amount Used |
| Iron(III) chloride hexahydrate | 0.1 g |
| Copper(II) chloride dihydrate | 0.005 g |
| Zinc chloride | 0.02 g |
| Magnesium(II) chloride tetrahydrate | 0.005 g |
| Sodium tetraborate decahydrate | 0.005 g |
| Ammonium molybdate tetrahydrate | 0.005 g |
| Water | Up to 100mL |

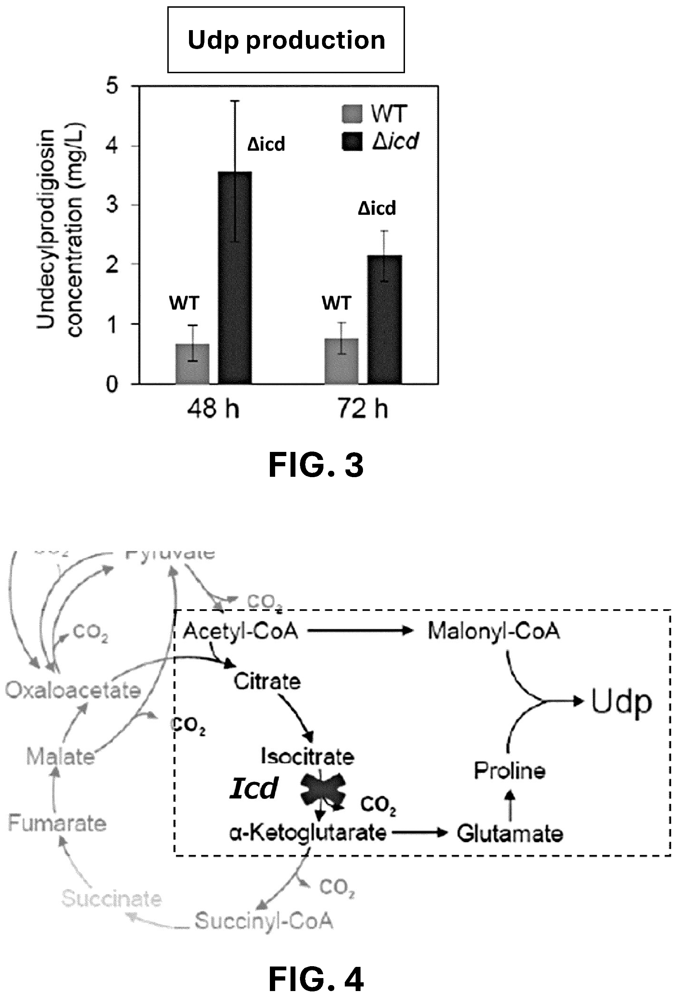

As the result is shown in FIG. 3, the Δicd strain produced slightly more than about 5 times Udp compared to the wild-type strain (WT). From this, it was revealed that reduction of the yield of CO₂ relative to carbon by icd disruption is effective for substance production.

### 3-2: Udp production of ΔpckA strain

The wild-type strain and the ΔpckA strain were each cultivated, and the production amount of Udp was confirmed. Each strain was cultivated on a solid TSB medium (30°C, 48 hours), and pre-cultured in 10 mL of R2YE medium (see Table 5 and Table 6) with shaking (28°C, 160 rpm, 48 hours). The whole amount was added to 50 mL of R2YE medium, and main culture (28°C, 160 rpm, 72 hours) was performed. 10 mL of culture was recovered 48 hours and 72 hours after the start of main culture, respectively, and centrifuged to recover bacterial cells. 5 mL of methanol was added to treat the bacterial cells with methanol, and Udp was extracted. The absorbance (530 nm) of the supernatant containing extracted Udp was measured, and the Udp concentration was calculated.

As the result is shown in FIG. 6, the ΔpckA strain produced about 2.1 times Udp at 48 hours and about 1.3 times Udp at 72 hours compared to the wild-type strain (WT). From this, it was revealed that reduction of the yield of CO₂ relative to carbon by pckA disruption is effective for substance production.

### Example 4: Primary metabolite production by enzyme gene-disrupted strain

### 4-1: Ribulose and α-ketoglutarate production in Δicd strain and strain

For the wild-type strain, Δicd strain, and strain, aerobic jar fermenter cultivation was performed. The culture fluid was recovered at 88 hours of cultivation and centrifuged at 15,000 rpm to obtain a culture supernatant. The obtained culture fluid was filtered with a 0.2 µm filter and then subjected to HPLC analysis. HPLC analysis conditions for ribulose and α-ketoglutarate are as shown in Table 7 and Table 8, respectively.

**[Table 7]**

| Table 7: Ribulose analysis method | |
|---|---|
| Column | Inert-Sphere Sugar-9 µm (7.8 × 300mm) |
| | GL Sciences |
| Solvent | H₂O |
| Detector | RID |
| Flow rate | 1.0 ml/min |
| Retention time | Around 12.5 min |

**[Table 8]**

| Table 8: alpha-ketoglutarate analysis method | |
|---|---|
| Column | Rezex ROA-Organic Acid H⁺ 8µm (4.6×300mm) |
| | Phenomenex |
| Solvent | 0.1% formic acid |
| Detector | RID |
| Flow rate | 0.5 ml/min |
| Retention time | Around 9.5 min |

Regarding ribulose production, whereas the wild-type strain was 0.83 g/L, the Δicd strain was 1.90 g/L and the strain was 1.24 g/L, and the production amount improved by about 2 times and 1.5 times, respectively. Regarding α-ketoglutarate production, whereas the wild-type strain was 0.63 g/L, the Δicd strain was 32.0 g/L and the strain was 0.98 g/L, and the production amount improved by 50.7 times and 1.6 times, respectively. From this, it was revealed that reduction of the yield of CO₂ relative to carbon by icd disruption and maeA disruption is effective for substance production.

Udp is synthesized from malonyl-CoA and proline. It is considered that malonyl-CoA is produced from acetyl-CoA, upstream of the icd enzyme, and proline is produced via glutamate from α-ketoglutarate, downstream of the icd enzyme. In this cultivation, proline was added to the medium, and it is considered that the decrease in proline expected due to icd enzyme disruption could be compensated.

The gene-disrupted strains used in these examples are listed below. All experiments were performed under aerobic conditions.

**[Table 9]**

| Table 9: List of test strains | |
|---|---|
| Disrupted gene | Notation |
| Wild type | WT |
| SLI_7202 | Δ*icd* |
| SLI_5252 | Δ*pckA* |
| SLI_3297, SLI_5552 | Δ*maeA* |
| SLI_4134 | Δ*gnd* |

As described above, according to these examples, a technique for reducing carbon dioxide emission amount associated with cultivation of actinomycetes was discovered. Thereby, it can be expected to reduce the environmental load (particularly CO₂ emission amount) due to cultivation of actinomycetes. Also, according to these examples, by disrupting an enzyme responsible for a metabolic reaction producing CO₂, carbon utilization efficiency in bacterial cells was greatly improved, and it succeeded in changing bacterial cells to a state more suitable for substance production. As shown in Example 3, by disrupting a gene of an enzyme responsible for a metabolic reaction producing CO₂, the production amount of actually useful metabolites could be increased. Although it is expected to be effective in all microorganisms (particularly bacteria), it is considered highly effective particularly in microorganisms (particularly bacteria) with a high yield relative to carbon of carbon dioxide, and in such microorganisms (particularly bacteria), the path for a gene-modified strain with reduced environmental load and/or improved metabolite production amount, a cultivation method thereof, and metabolite production using these is considered to be opened.

## Claims

1. A metabolic enzyme gene-disrupted strain of an actinomycete whose yield relative to carbon of carbon dioxide (CO₂) exceeds 50% in at least a logarithmic growth phase or a stationary phase in aerobic culture in a medium containing a sufficient amount of a carbon source and other nutrients, wherein at least one of genes encoding an enzyme responsible for a metabolic reaction producing CO₂ is disrupted, and the yield of CO₂ relative to carbon is reduced relative to a control actinomycete of the same species in which none of the genes are disrupted.

2. The metabolic enzyme gene-disrupted strain according to claim 1, wherein the enzyme responsible for a metabolic reaction producing CO₂ is an enzyme of any one or more metabolic pathways selected from the group consisting of glycolysis, pentose phosphate pathway, and citric acid cycle.

3. The metabolic enzyme gene-disrupted strain according to claim 1 or 2, wherein one or more selected from the group consisting of phosphogluconate dehydrogenase, pyruvate dehydrogenase, phosphoenolpyruvate carboxykinase, malate dehydrogenase, isocitrate dehydrogenase, dihydrolipoyl transacetylase, dihydrolipoyl dehydrogenase, and α-ketoglutarate dehydrogenase are disrupted.

4. The metabolic enzyme gene-disrupted strain according to any one of claims 1 to 3, wherein in aerobic culture in the presence of a sufficient amount of a carbon source, compared to the control actinomycete, its growth ability is equivalent, or it does not show a decrease in growth ability by 50% or more.

5. The metabolic enzyme gene-disrupted strain according to any one of claims 1 to 4, wherein one or more genes consisting of at least isocitrate dehydrogenase, phosphogluconate dehydrogenase, phosphoenolpyruvate carboxykinase, and malate dehydrogenase are disrupted.

6. The metabolic enzyme gene-disrupted strain according to any one of claims 1 to 5, wherein the actinomycete is of the genus Streptomyces.

7. A composition comprising the metabolic enzyme gene-disrupted strain according to any one of claims 1 to 6, for use in producing a metabolite.

8. A composition comprising the metabolic enzyme gene-disrupted strain according to any one of claims 1 to 6, for use in producing a metabolite.

9. A method for cultivating a metabolic enzyme gene-disrupted strain of an actinomycete, wherein
the metabolic enzyme gene-disrupted strain comprises the metabolic enzyme gene-disrupted strain according to any one of claims 1 to 6, and
the method comprises cultivating the metabolic enzyme gene-disrupted strain in a medium containing a sufficient amount of a carbon source and other nutrients under aerobic conditions.

10. The method according to claim 9, wherein the cultivation comprises cultivating the metabolic enzyme gene-disrupted strain in a logarithmic growth phase, and cultivating the metabolic enzyme gene-disrupted strain in a stationary phase.

11. A method for producing a metabolite using a metabolic enzyme gene-disrupted strain of an actinomycete, comprising:
cultivating a metabolic enzyme gene-disrupted strain, wherein
the metabolic enzyme gene-disrupted strain comprises the metabolic enzyme gene-disrupted strain according to any one of claims 1 to 6,
the method comprises cultivating the metabolic enzyme gene-disrupted strain in a medium containing a sufficient amount of a carbon source and other nutrients under aerobic conditions to cause the metabolic enzyme gene-disrupted strain to produce the metabolite; and
recovering the metabolite.

12. The method according to claim 11, wherein the metabolite is derived from the citric acid cycle.

13. A method for selecting an actinomycete, comprising:
providing an isolated actinomycete;
measuring a yield relative to carbon of carbon dioxide of the actinomycete in a logarithmic growth phase or a stationary phase in aerobic culture in a medium containing a sufficient amount of a carbon source and other nutrients; and
selecting or obtaining an actinomycete whose yield relative to carbon exceeds 50% in either the logarithmic growth phase or the stationary phase.

14. A method for genetically modifying an actinomycete to obtain a genetically modified actinomycete, comprising:
providing an isolated actinomycete;
measuring a yield relative to carbon of carbon dioxide of the actinomycete in a logarithmic growth phase or a stationary phase in aerobic culture in a medium containing a sufficient amount of a carbon source and other nutrients;
selecting or obtaining an actinomycete whose yield relative to carbon exceeds 50% in either the logarithmic growth phase or the stationary phase;
disrupting by genetic modification at least one of genes encoding an enzyme responsible for a metabolic reaction producing CO₂ of the obtained actinomycete; and
obtaining the genetically modified actinomycete.
